# EUROPÄISCHE PATENTANMELDUNG

(11) **EP 2 436 422 A1**
(43) Veröffentlichungstag der Anmeldung: **04.04.2012**
(21) Anmeldenummer: 11179128.1
(22) Anmeldetag: 29.08.2011
(51) Int. Cl.: A61N 1/05, A61N 1/08, A61N 1/37

(54) **Implantierbarer Hydrogelsensor**

(30) Priorität: 23.09.2010 US 385565 P
(71) Anmelder: BIOTRONIK SE & Co. KG, 12359 Berlin (DE)
(72) Erfinder: Dörr, Thomas, 12437 Berlin (DE)
(74) Vertreter: Lindner-Vogt, Karin L.

(57) **Zusammenfassung**

Die Erfindung betrifft einen implantierbarer Hydrogelsensor, welcher eine Hydrogelkapsel umfasst, wobei die Hydrogelkapsel eine wasserdurchlässige Wand hat, die einen Hohlraum formt, in dem ein Hydrogel mit einem temperaturabhängigen Quellvermögen enthalten ist, wobei die Wand der Hydrogelkapsel so ausgebildet ist, dass sie sich dem Volumen des Hydrogels anpassen kann.

## Beschreibung

Die Erfindung liegt auf dem Gebiet der Medizintechnik und betrifft eine implantierbare Elektrodenleitung nach dem Oberbegriff von Patentanspruch 1 sowie eine Elektrodenleitungsanordnung nach dem Oberbegriff von Patentanspruch 8. Weiterhin erstreckt sich die Erfindung auf einen implantierbaren Hydrogelsensor, und dessen Verwendung. Ferner erstreckt sich die Erfindung auf ein Verfahren zum Bestimmen der Temperatur der Elektrode einer erfindungsgemäßen Elektrodenleitung, ein Verfahren zum Betreiben eines Kernspintomographen und dessen Verwendung.

Gattungsgemäße Elektrodenleitungen sind in mannigfachen Ausführungen bekannt und werden beispielsweise im Bereich der Elektrophysiologie zur Erfassung und Behandlung von Reizleitungsstörungen im Herzen oder im Nervensystem routinemäßig eingesetzt. Je nach Anwendung werden sie beispielsweise auch als Stimulationselektrode, Herzschrittmacherelektrode, ICD-Elektrode (ICD = Implantierbarer Cardioverter) oder Elektrophysiologie-Katheter bezeichnet. In der Patentliteratur wurden sie bereits vielfach beschrieben. Lediglich beispielhaft sei in diesem Zusammenhang auf die Druckschriften DE 10 2005 039 040 A1, DE 198 00 697 Al, DE 20 2006 020 517 U1 und EP 0 306 442 verwiesen.

Wie dem Fachmann an sich bekannt ist, können im Körper eines Patienten befindliche Elektrodenleitungen Probleme bei der Untersuchung mittels eines Kernspintomographen verursachen. Bei der Kernspin- oder Magnetresonanztomographie (MRT = Magnetresonanztomographie) werden Atomkerne durch starke statische Magnetfelder zu einer Präzessionsbewegung um die Feldrichtung gezwungen und durch hochfrequente elektromagnetische Wechselfelder resonant angeregt. Nach Abschalten der Wechselfelder kehren die Atomkerne wieder in ihre Ausgangsrichtung zurück, wobei die Abklinkzeiten (Relaxationszeiten) charakteristisch für verschiedene Gewebearten sind, so dass eine genaue Bildgebung ermöglicht ist.

Nun besteht bei einem Patienten mit einer implantierten Elektrodenleitung die Gefahr, dass sich deren Elektroden und die umgebenden Gewebsschichten erwärmen. Ursache hierfür sind elektrische Induktionsströme, die durch die starken elektromagnetischen Wechselfelder des Kernspintomographen erzeugt werden. Aus diesem Grund muss der diagnostische Nutzen einer kernspintomographischen Untersuchung gegenüber dem Risiko nachteiliger Nebenwirkungen sorgfältig abgewogen werden. Erschwerend kommt hinzu, dass die Erwärmung der Elektroden von den jeweiligen Scan-Parametern der Untersuchung und insbesondere von der Positionierung der Elektrodenleitung im elektromagnetischen Wechselfeld abhängen, so dass in der klinischen Praxis oftmals keine verlässlichen Vorhersagen getroffen werden können. Dies führt in der Regel dazu, dass Patienten mit einer implantierten Elektrodenleitung von der an sich schonenden Untersuchung mittels Kernspintomographie ausgeschlossen sind.

Demgegenüber besteht die Aufgabe der vorliegenden Erfindung darin, eine implantierbare Elektrodenleitung für die medizinische Anwendung zur Verfügung zu stellen, welche eine Untersuchung mittels Kernspintomographie zuverlässig und sicher ermöglicht. Zudem soll eine solche Elektrodenleitung einfach einsetzbar und in der Serienfertigung kostengünstig herstellbar sein. Diese und weitere Aufgaben werden nach dem Vorschlag der Erfindung durch eine Elektrodenleitung und eine Elektrodenleitungsanordnung mit den Merkmalen der unabhängigen Patentansprüche 1 und 8 gelöst. Vorteilhafte Ausgestaltungen der Erfindung sind durch die Merkmale der Unteransprüche angegeben.

Erfindungsgemäß ist eine implantierbare Elektrodenleitung zur medizinischen Anwendung gezeigt. Wie hier verwendet, bezeichnet der Ausdruck "implantierbar" einen dauerhaften oder nur kurzzeitigen, vorübergehenden Verbleib im Körper eines Patienten, beispielsweise nur während einer Untersuchung. Die Elektrodenleitung kann in mannigfachen Ausgestaltungen vorliegen, wobei es sich beispielsweise um eine Stimulationselektrode, insbesondere zur Nervenstimulation, eine Herzschrittmacherelektrode, eine ICD-Elektrode, einen Elektrophysiologie-Katheter oder eine Elektrode zur Messung von Hirnpotentialen oder dergleichen handeln kann. Selbstverständlich ist diese Aufzählung nicht abschließend.

Die Elektrodenleitung umfasst gattungsgemäß einen distalen Abschnitt mit zumindest einem elektrisch leitenden Oberflächenbereich zur Aufnahme elektrischer Signale oder zur Abgabe elektrischer Impulse, d. h. zur Übertragung von elektrischer Energie zwischen der Elektrodenleitung und deren Umgebung. Hier und im Weiteren wird der elektrisch leitende Oberflächenbereich als "Elektrode" bezeichnet. Eine andere übliche Bezeichnung hierfür wäre "Pol". Die zumindest eine Elektrode der Elektrodenleitung ist mit einer elektrischen Zuleitung verbunden, die an einem proximalen Ende der Elektrodenleitung mit einem elektrischen Gerät, beispielsweise einem Herzschrittmacher, elektrisch verbunden werden kann. Die Oberfläche der Elektrodenleitung ist mit Ausnahme der zumindest einen Elektrode in der Regel elektrisch isoliert.

Gemäß vorliegender Erfindung zeichnet sich die Elektrodenleitung in wesentlicher Weise dadurch aus, dass die Elektrodenleitung über zumindest eine mit der Elektrode thermisch gekoppelte Hydrogelkapsel verfügt, wobei die Hydrogelkapsel eine zumindest abschnittsweise wasserdurchlässige Wand hat, die einen Hohlraum formt, in dem ein Hydrogel mit einem temperaturabhängigen Quellvermögen enthalten ist. Dabei ist die Wand der Hydrogelkapsel so ausgebildet, dass sie sich dem vom Wassergehalt abhängigen Volumen des Hydrogels anpassen kann. Dies bedeutet, dass sich die Hydrogelkapsel mit einer Volumenzunahme des Hydrogels vergrößert und mit einer Volumenabnahme des Hydrogels verkleinert, ohne die Volumenänderung des Hydrogels nennenswert zu beeinträchtigen. Die Wand der Hydrogelkapsel besteht zu diesem Zweck beispielsweise aus einem elastisch verformbaren Material, dessen Elastizitätsmodul eine für diesen Zweck geeignete Größe hat. Die Hydrogelkapsel bzw. das darin enthaltene Hydrogel ist mit der Elektrode thermisch gekoppelt, so dass die Temperatur des Hydrogels einer Temperaturänderung der Elektrode folgen kann. Die Wand der Hydrogelkapsel verfügt zu diesem Zweck über eine hinreichende Wärmeleitfähigkeit. Falls die Elektrodenleitung über zwei oder mehr Elektroden verfügt, ist es bevorzugt, wenn jede Elektrode mit einer separaten Hydrogelkapsel thermisch gekoppelt ist.

Mit zumindest abschnittsweise ist gemeint, dass auch nur ein Teil der Wand oder die komplette Wand der Hydrogelkapsel wasserdurchlässig ausgebildet ist.

So ist in einer speziellen Ausführungsform nur der distale Boden der Hydrogelkapsel wasserdurchlässig.

In einer weiteren Ausführungsform beinhaltet die Elektrodenleitung bzw. der eingesetzte Führungsdraht selbst ein Wasserdepot (720), dass mit einer Membrane (730) oder Zuleitung mit der Hydrogelkapsel (710) verbunden ist und so die wasserdurchlässige Wand bildet, während der Rest der Hydrogelkapsel wasserundurchlässig ist. So kann abhängig von der Erwärmung der Wassergehalt zwischen Hydrogelkapsel und Wasserdepot (740) verschoben werden und per MRT-Bildauswertung das Verhältnis des Wassergehaltes entsprechend ausgewertet werden. Diese Methode bietet den Vorteil, dass dies auch in einem von der Körperflüssigkeit isoliertem Bauteil wie z.B. dem Führungsdraht eingesetzt werden kann und dass durch die Auswertung des Verhältnisses des Wasseranteils keine oder eine deutlich vereinfachte Kalibrierung benötigt wird.

In Einklang mit der üblichen Verwendung des Begriffs Hydrogel handelt es sich hierbei um eine Substanz, die Wasser binden kann, jedoch selbst im Wasser unlöslich ist. In der Regel sind dies hydrophile Polymere, deren Ketten durch kovalente Bindungen, ionische Bindungen und/oder physikalische Wechselwirkungen zu einem dreidimensionalen Netzwerk verbunden sind. Durch die hydrophilen Bestandteile sind Hydrogele in der Lage, Wassermoleküle reversibel zu binden. In der vorliegenden Erfindung kommen ausschließlich solche Hydrogele zur Anwendung, die ein temperaturabhängiges Quellvermögen haben. Dies bedeutet, dass sich der Wassergehalt und somit das Volumen des Hydrogels mit einer Änderung der Temperatur des Hydrogels ändert.

Die erfindungsgemäße Elektrodenleitung ermöglicht in vorteilhafter Weise eine einfache und sichere Bestimmung der Temperatur der thermisch gekoppelten Elektrode, indem eine äußere Abmessung bzw. das Volumen der Hydrogelkapsel gemessen wird. Ein besonderer Vorteil der Erfindung ergibt sich aus der Tatsache, dass das Hydrogel in einer kernspintomographischen Aufnahme aufgrund des sehr hohen Wassergehalts gut erkennbar ist, so dass die äußere Abmessung der Hydrogelkapsel in einfacher Weise in der kernspintomographischen Aufnahme ausgewertet werden kann. Die Wandstärke der Kapsel ist in der Regel vernachlässigbar gering gegenüber der Ausdehnung des Hydrogels. Somit kann insbesondere während einer kernspintomographischen Untersuchung die Temperatur der thermisch gekoppelten Elektrode der Elektrodenleitung laufend überwacht werden. Das in der Hydrogelkapsel enthaltene Hydrogel dient auf diese Weise als Sensor zum Erfassen der Temperatur der Elektrode der Elektrodenleitung.

Bei einer vorteilhaften Ausführungsform der erfindungsgemäßen Elektrodenleitung befindet sich die das Hydrogel enthaltende Hydrogelkapsel in Nähe der Elektrode bzw. grenzt an diese an, um auf diese Weise eine gute thermische Kopplung zwischen Elektrode und Hydrogel zu erreichen. Vorzugsweise befindet sich die Hydrogelkapsel in Berührungskontakt mit der Elektrode um einen effizienten Wärmeübergang durch Wärmeleitung zu ermöglichen.

Vorzugsweise handelt es sich bei der Elektrode um eine an einem distalen Ende der Elektrodenleitung angeordnete Spitzen- bzw. Tip-Elektrode, da in diesem Fall einer Kontrolle der Elektrodentemperatur aufgrund der kleinen Elektrodenfläche und der damit einher gehenden hohen Gefahr einer Gewebsschädigung besonders wichtig ist.

Wie bereits ausgeführt wurde, ist das Quellvermögen des Hydrogels von dessen Temperatur abhängig. Bei einer vorteilhaften Ausführungsform der erfindungsgemäßen Elektrodenleitung verringert sich das Quellvermögen des Hydrogels bei steigender Temperatur der Hydrogels, so dass sich der Wassergehalt und somit das Volumen des Hydrogels verringert, wenn die Temperatur der Elektrode ansteigt. Andererseits erhöht sich das Quellvermögen des Hydrogels bei fallender Temperatur des Hydrogels, so dass sich der Wassergehalt und somit das Volumen des Hydrogels vergrößert, wenn die Temperatur der Elektrode fällt. Bevorzugt ist das Hydrogel so gewählt, dass sich das Quellvermögen des Hydrogels bei einem Temperaturanstieg des Hydrogels von 10°C um mindestens 30% verringert, wodurch in der Praxis häufig auftretende Temperaturänderungen der Elektroden zuverlässig und sicher erfasst werden können. Grundsätzlich kann das Hydrogel so gewählt sein, dass die abhängig von der jeweiligen Anwendung typischer Weise auftretenden Temperaturänderungen der thermisch gekoppelten Elektrode mit einer hohen Volumenänderung des Hydrogels bzw. Hydrogelkapsel einhergehen.

Bei dem Hydrogel handelt es vorzugsweise um ein Polymer, das aus einer oder mehreren Substanzen, gewählt aus der Gruppe, bestehend aus Acrylamid, Metacrylamid, Dimethylaminoethylmethacrylat und einem Derivat dieser Substanzen, hergestellt ist bzw. hierauf basiert. Gleichermaßen kann es bevorzugt sein, wenn das Hydrogel aus Poly(N-isopropylacrylamid) und/oder Poly(N-isopropylacrylamid-coallylamin) hergestellt ist oder hierauf basiert. Besonders bevorzugt handelt es sich bei dem Hydrogel um Poly(N-isopropylacrylamid) bzw. PNIPAM mit Poly(P-dioxanon) als Hartsegment.

Die Wand der das Hydrogel enthaltenden Hydrogelkapsel besteht vorzugsweise aus einem Polyester, insbesondere aus einer oder mehreren Substanzen, gewählt aus der Gruppe, bestehend aus Poly-L-Lactid (PLLA), Poly-DL-Lactid (PDLLA), Poly-Lactid-Co-Glycolid (PLGA), Poly-3-hydroxybutyrat (P3HB), Poly-4-hydroxybutyrat (P4HB), Derivaten und Copolymeren hiervon. Bevorzugt besteht die Wand der Hydrogelkapsel aus Parylen, insbesondere Parylen C oder andere Derivate, besonders bevorzugt Parylen mit kleinen Löchern (Pin Holes).

Bei einer weiteren vorteilhaften Ausführungsform der erfindungsgemäßen Elektrodenleitung ist die Hydrogelkapsel in der Elektrodenleitung vollständig aufgenommen, wobei die Wand der Hydrogelkapsel einen Oberflächenbereich der Elektrodenleitung bildet, so dass die Handhabung der Elektrodenleitung durch die Hydrogelkapsel nicht beeinträchtigt ist.

Bei einer weiteren vorteilhaften Ausführungsform der erfindungsgemäßen Elektrodenleitung ist die Hydrogelkapsel und das darin enthaltene Hydrogel mit einem Durchgang zum Durchführen eines Führungsmittels für die Elektrodenleitung versehen, so dass die Handhabung der Elektrodenleitung durch die Hydrogelkapsel nicht beeinträchtigt ist und die Elektrodenleitung in herkömmlicher Weise verlegt werden kann.

Die Erfindung erstreckt sich weiterhin auf eine Elektrodenleitungsanordnung zur medizinischen Anwendung, welche eine implantierbare Elektrodenleitung mit einem distalen Abschnitt mit zumindest einer Elektrode zur Übertragung von elektrischer Energie zwischen der Elektrodenleitung und deren Umgebung sowie ein in die Elektrodenleitung einführbares Führungsmittel zum Führen der Elektrodenleitung umfasst.

Die Elektrodenleitung zeichnet sich in wesentlicher Weise dadurch aus, dass das Führungsmittel über zumindest eine Hydrogelkapsel verfügt, die so angeordnet ist, dass sie in einer zum Führen der Elektrodenleitung geeigneten Stellung des Führungsmittels mit der Elektrode thermisch gekoppelt ist. Die Hydrogelkapsel ist mit einer wasserdurchlässigen Wand versehen, die einen Hohlraum formt, in dem ein Hydrogel mit einem temperaturabhängigen Quellvermögen enthalten ist, wobei die Wand so ausgebildet ist, dass sie sich dem Volumen des Hydrogels anpassen kann. Bezüglich des Hydrogels und der Hydrogelkapsel wird zur Vermeidung unnötiger Wiederholungen auf obige Ausführungen Bezug genommen.

In einer besonders bevorzugten Ausführungsform ist die Hydrogelkapsel mit einem Wasserreservoir über eine wasserdurchlässige Wand, wie eine Membran oder eine Leitung oder Öffnung, verbunden. Die nicht an das Wasserreservoir angrenzenden Wände können entweder wasserdurchlässig oder wasserundurchlässig sein. Durch das Wasserreservoir wird die Meßmethode mit der Hydrogelkapsel unabhängig von einem Kontakt mit Körperflüssigkeit.

Die Erfindung erstreckt sich weiterhin auf einen implantierbaren Hydrogelsensor, welcher eine Hydrogelkapsel mit einer wasserdurchlässigen Wand umfasst, die einen Hohlraum formt, in dem ein Hydrogel mit einem temperaturabhängigen Quellvermögen enthalten ist. Dabei ist die Wand so ausgebildet, dass sie sich dem Volumen des Hydrogels anpassen kann. Bezüglich des Hydrogels und dessen Wand wird zur Vermeidung unnötiger Wiederholungen auf obige Ausführungen Bezug genommen. Eine äußere Abmessung des Hydrogelsensors verändert sich somit durch Aufnahme oder Abgabe von Wasser in Abhängigkeit von der Umgebungstemperatur des Hydrogelsensors. Das verwendete Hydrogel kann in Abhängigkeit von den bei der jeweiligen Anwendung typischer Weise auftretenden Änderungen der Umgebungstemperatur spezifisch gewählt sein, so dass die Temperaturänderungen mit einer großen Volumenänderung des Hydrogelsensors einhergehen. Wie bereits ausgeführt, kann die Größe der Hydrogelkapsel in einfacher Weise durch Auswertung einer kernspintomographischen Aufnahme bestimmt werden.

Die Erfindung erstreckt sich weiterhin auf die Verwendung eines erfindungsgemäßen, implantierbaren Hydrogelsensors zur Bestimmung der Umgebungstemperatur des implantierten Hydrogelsensors.

Bevorzugt wird auch ein Verfahren zum Bestimmen der Umgebungstemperatur eines erfindungsgemäßen, implantierbaren Hydrogelsensors. Hierbei wird zunächst ein Hydrogelsensor bereitgestellt, anschließend eine äußere Abmessung der Hydrogelkapsel gemessen und die Temperatur der Umgebung der Hydrogelkapsel auf Basis der äußeren Abmessung der Hydrogelkapsel ermittelt.

Die Erfindung erstreckt sich weiterhin auf ein Verfahren zum Bestimmen der Temperatur einer Elektrode einer erfindungsgemäßen, implantierbaren Elektrodenleitung. Hierbei wird zunächst eine Elektrodenleitung bereitgestellt, anschließend eine äußere Abmessung der zumindest einen Hydrogelkapsel gemessen und die Temperatur der thermisch gekoppelten Elektrode auf Basis der äußeren Abmessung der Hydrogelkapsel ermittelt. Insbesondere kann hierbei auf Basis einer Änderung der äußeren Abmessung der Hydrogelkapsel eine Änderung der Temperatur der Elektrode bestimmt werden. Vorzugweise wird die äußere Abmessung der Hydrogelkapsel durch Auswertung einer kernspintomographischen Aufnahme bestimmt.

Die Erfindung erstreckt sich weiterhin auf ein Verfahren zum Positionieren einer Elektrodenleitung, bei welchem zunächst eine erfindungsgemäße Elektrodenleitungsanordnung bereitgestellt wird, wobei das Führungsmittel in eine zum Führen der Elektrodenleitung geeignete Stellung gebracht wird, in der die Hydrogelkapsel mit der Elektrode thermisch gekoppelt ist. Anschließend wird während des Führens der Elektrodenleitung durch das Führungsmittel eine äußere Abmessung der Hydrogelkapsel gemessen und die Temperatur der thermisch gekoppelten Elektrode auf Basis der äußeren Abmessung der Hydrogelkapsel ermittelt. Vorzugweise wird die äußere Abmessung der Hydrogelkapsel durch Auswertung einer kernspintomographischen Aufnahme bestimmt. Nach Positionierung der Elektrodenleitung kann das Führungsmittel wieder entfernt werden.

Die Erfindung erstreckt sich weiterhin auf ein Verfahren zum Betreiben eines Kernspintomographen, bei welchem zunächst eine erfindungsgemäße, implantierbare Elektrodenleitung bereitgestellt wird, anschließend eine äußere Abmessung der Hydrogelkapsel gemessen und die Temperatur der thermisch gekoppelten Elektrode auf Basis der äußeren Abmessung der Hydrogelkapsel ermittelt wird. Vorzugweise wird die äußere Abmessung der Hydrogelkapsel durch Auswertung einer kernspintomographischen Aufnahme bestimmt. Auf Basis der Temperatur der Elektrode werden anschließend ein oder mehrere der folgenden Schritte ausgeführt:
- Reduzierung der Intensität von zur resonanten Anregung von Atomkernen eingesetzten elektromagnetischen Wechselfeldern, falls die Temperatur der Elektrode einen vorbestimmbaren ersten Schwellwert übersteigt;
- Abschalten der elektromagnetischen Wechselfelder, falls die Temperatur der Elektrode einen vorbestimmbaren zweiten Temperaturschwellwert übersteigt, welcher identisch zum ersten Temperaturschwellwert sein kann;
- Darstellen der Temperatur der Elektrode in einer graphischen Anzeigeeinrichtung des Kernspintomographen;
- Beaufschlagen der Elektrode mit einem elektrischen Impuls zum Zwecke einer Ablation umliegenden Körpergewebes.

Die Erfindung erstreckt sich weiterhin auf die Verwendung eines Kernspintomographen zum Bestimmen einer äußeren Abmessung einer mit einer Elektrode einer erfindungsgemäßen, implantierbaren Elektrodenleitung thermisch gekoppelten Hydrogelkapsel.

Es versteht sich, dass die verschiedenen Ausführungsformen und Ausgestaltungen der erfindungsgemäßen Gegenstände einzeln oder in beliebigen Kombinationen realisiert sein können. Insbesondere sind die vorstehend genannten und nachstehend zu erläuternden Merkmale nicht nur in den angegebenen Kombinationen, sondern auch in anderen Kombinationen oder in Alleinstellung einsetzbar, ohne den Rahmen der vorliegenden Erfindung zu verlassen.

Die Erfindung wird nun anhand von Ausführungsbeispielen näher erläutert, wobei Bezug auf die beigefügten Zeichnungen genommen wird. Gleiche bzw. gleich wirkende Elemente sind mit denselben Bezugszahlen bezeichnet. Es zeigen:
- Fig. 1: eine schematische Darstellung zur Veranschaulichung eines Ausführungsbeispiels der erfindungsgemäßen Elektrodenleitung;
- Fig. 2: eine vergrößerte Ansicht des distalen Abschnitts der Elektrodenleitung von Fig. 1;
- Fig. 3A-3B: schematische Darstellungen zur Veranschaulichung eines Ausführungsbeispiels der erfindungsgemäßen Elektrodenleitungsanordnung;
- Fig.4: eine schematische Darstellung zur Veranschaulichung eines Ausführungsbeispiels des erfindungsgemäßen Hydrogelsensors;
- Fig. 5: eine schematische Darstellung zur Veranschaulichung einer herkömmlichen, dauerhaft implantierten Elektrodenleitung.
- Fig. 6: eine Schematische Darstellung einer Hydrogelkapsel in Kombination mit einem Wasserreservoir und einer Wasserdurchlässigen Verbindung/Wand zwischen Wasserreservoir und Hydrogelkapsel.

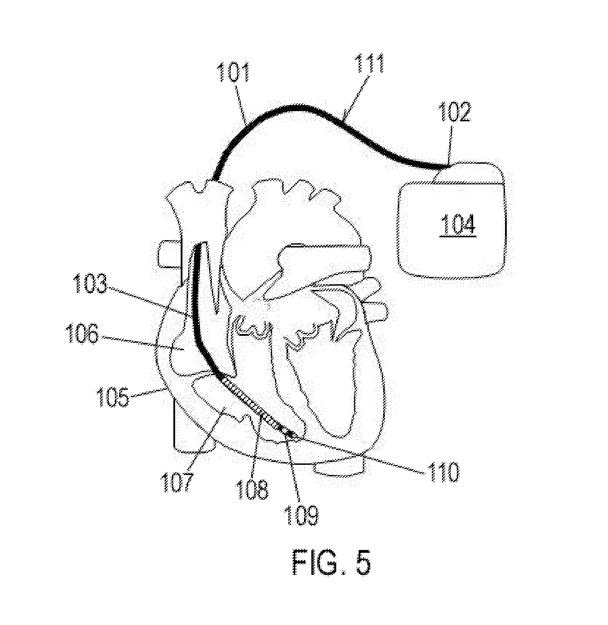

Sei zunächst Fig. 5 betrachtet, worin anhand einer schematischen Darstellung eine im Stand der Technik wohlbekannte, in einem Herz 105 dauerhaft implantierte Elektrodenleitung 101 veranschaulicht ist. Die Elektrodenleitung 101 ist hier beispielsweise als Schockelektrodenleitung ausgebildet. Sie verfügt über einen distalen Abschnitt 103 und ein proximales Ende 102, das mit einem implantierten ICD 104 (Implantierbarer Kardioverter/Defibrillator) verbunden ist. Hierbei handelt es sich um ein Gerät, welches Schockimpulse erzeugen kann, beispielsweise um ein lebensbedrohliches Kammerflimmern zu beenden. Die Elektrodenleitung 101 passiert durch den rechten Herzvorhof 106 und ist mit ihrem distalen Abschnitt 103 innerhalb der rechten Herzkammer 107 positioniert. Der distale Abschnitt 103 ist mit einer in Form eines wendelförmigen Drahts ausgebildeten Schockelektrode 108 versehen, die in Kontakt mit der Wand der Herzkammer 107 ist. Die längliche Wendelform der Schockelektrode 108 dient für eine Vergrößerung der effektiven Elektrodenfläche. Der distale Abschnitt 103 weist weiterhin eine Ringelektrode 109 und eine konusförmige Spitzenelektrode 110 auf, die für Abtast- und/oder Stimulationszwecke (antibradykarde Stimulation) eingesetzt werden können. Eine Oberfläche 111 der Elektrodenleitung 101 ist mit Ausnahme der Elektroden 108-110, welche elektrisch leitende Oberflächenbereiche zur Übertragung von elektrischer Energie darstellen, elektrisch isoliert.

Bei einer kernspintomographischen Untersuchung besteht nun vor allem für die kleinflächige Spitzenelektrode 110 die Gefahr einer Erwärmung durch die dabei eingesetzten elektromagnetischen Wechselfelder. Diese Erwärmung kann gegebenenfalls zu einem Anstieg der Stimulationsreizschwellen, einer Beeinträchtigung der Wahrnehmungsfunktion und/oder zu einer dauerhaften Schädigung des umliegenden Herzmuskelgewebes führen. In der Folge kann eine Elektrodendislokation und/oder Herzmuskelperforation auftreten.

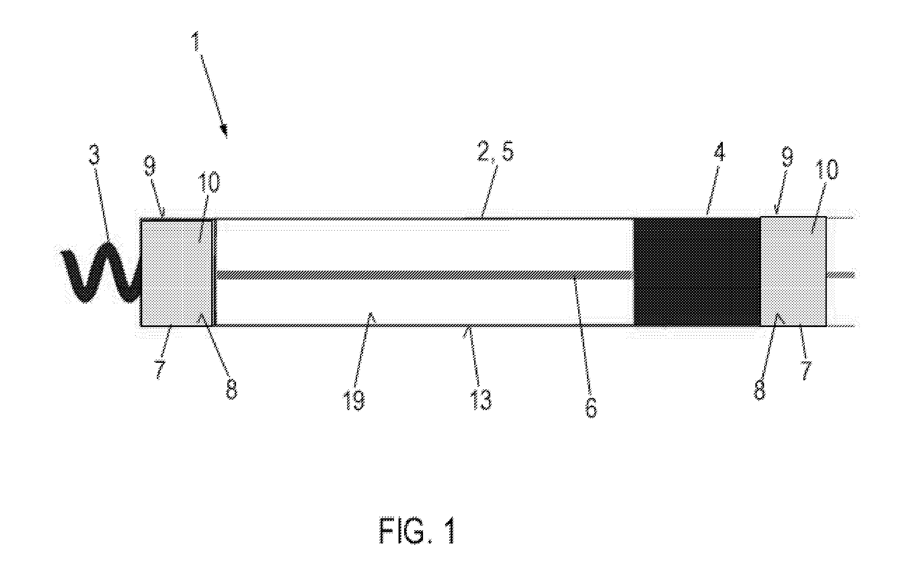

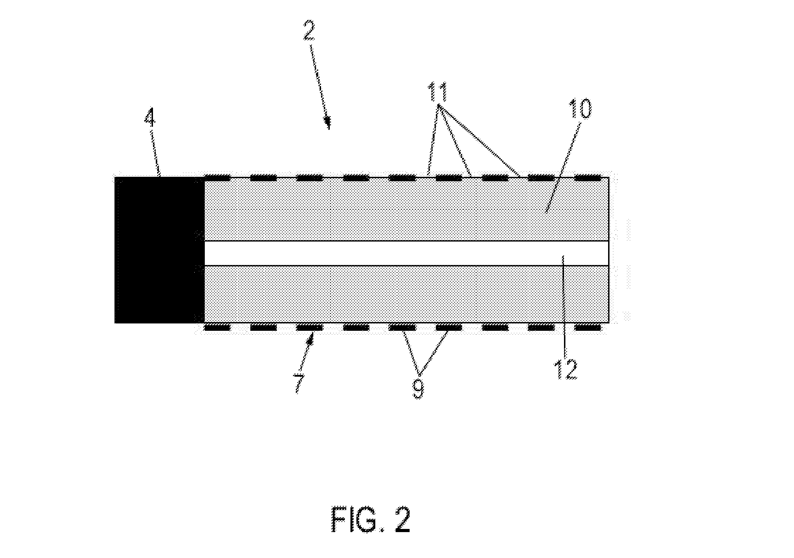

Es wird nun Bezug auf Fig. 1 und Fig. 2 genommen, worin ein Ausführungsbeispiel der erfindungsgemäßen Elektrodenleitung 1 veranschaulicht ist. Die Elektrodenleitung 1 ist hier beispielsweise als implantierbare Schrittmacherelektrodenleitung ausgebildet. Sie kann analog zu der in Fig. 5 veranschaulichten Weise im Körper eines Patienten implantiert werden, wobei anstelle eines ICD 104 ein Herzschrittmacher (nicht gezeigt) an die Elektrodenleitung 1 anzuschließen ist.

Sei zunächst Fig. 1 betrachtet, worin ein distaler Abschnitt 2 der Elektrodenleitung 1 gezeigt ist. Demnach umfasst die Elektrodenleitung 1 einen schlauchförmigen, flexiblen Leitungskörper 5, der einen Leitungskörperhohlraum 19 formt. Der Leitungskörper 5 ist mit einer hier beispielsweise in Form einer Einschraubelektrode zum Einschrauben in das Herzgewebe ausgebildeten Spitzenelektrode 3 und einer proximal versetzten Ringelektrode 4 versehen. Sowohl die Spitzenelektrode 3 als auch die Ringelektrode 4 sind mit einer innerhalb des Leitungskörperhohlraums 19 angeordneten elektrischen Zuleitung 6 (Leiterseele) verbunden, welche am proximalen Ende der Elektrodenleitung 1 an den Herzschrittmacher angeschlossen werden kann. Die Spitzenelektrode 3 kann durch Drehen der elektrischen Zuleitung 6 gedreht werden, um hierdurch eine dauerhafte Verankerung der Elektrodenleitung 1 im Herzmuskelgewebe zu erreichen. Die Verankerung einer Elektrodenleitung mittels Einschraubelektrode ist dem Fachmann an sich bekannt, beispielsweise aus der eingangs genannten Druckschrift DE 20 2006 020 517 U1, so dass hier nicht näher darauf eingegangen werden muss.

Weiterhin umfasst die Elektrodenleitung 1 zwei Hydrogelkapseln 7, von denen eine mit der Spitzenelektrode 3 und die andere mit der Ringelektrode 4 thermisch gekoppelt ist. Beide Hydrogelkapseln 7 grenzen unmittelbar an die mit ihnen thermisch gekoppelten Elektroden 3, 4 an und sind mit diesen jeweils in Berührungskontakt. Die beiden Hydrogelkapseln 7 sind jeweils vollständig im Leitungskörperhohlraum 19 aufgenommen. Die elektrische Zuleitung 6 ist durch die beiden Hydrogelkapseln 7 hindurch geführt.

Jede Hydrogelkapsel 7 umfasst eine Wand 9 aus einem elastisch nachgiebigen Material, welche ein Kapselhohlraum 8 formt, in dem ein Hydrogel 10 enthalten ist. Das Hydrogel 10 besteht aus einem dreidimensionalen hydrophilen Polymer, wobei es sich im vorliegenden Ausführungsbeispiel beispielsweise um Poly(N-isopropylacrylamid) mit Poly(P-dioxanon) als Hartsegment handelt. Das Quellvermögen des Hydrogels 10 ist temperaturabhängig, wobei sich das Quellvermögen mit steigender Temperatur verringert. Das Quellvermögen des Hydrogels 10 verringert sich bei einem Temperaturanstieg von 10°C um mindestens 30%. Die Wände 9 der beiden Hydrogelkapseln 7 sind so nachgiebig, dass sie sich dem vom Wassergehalt abhängigen Volumen des enthaltenen Hydrogels 10 anpassen können. Zu diesem Zweck sind Wände 9 jeweils aus einem Polyester mit einer geeigneten Wandstärke gefertigt, wobei sie im vorliegenden Ausführungsbeispiel aus Parylen C mit einer Vielzahl kleiner Löcher (Pin Holes) 11 bestehen. Die kleinen Löcher 11 halten das Hydrogel 10 zurück, lassen jedoch Wasser hindurch treten, so dass sich der Wassergehalt des Hydrogels 10 in Abhängigkeit von dessen Temperatur ändern kann. Die beiden Hydrogelkapseln 7 sind jeweils mit einem Durchgang 12 zum Durchführen eines Führungsdrahts (nicht dargestellt) für die Elektrodenleitung 1 versehen.

Die Spitzen- und Ringelektrode 3, 4 stellen elektrisch leitende Bereiche einer ansonsten elektrisch isolierten Oberfläche 13 des Leitungskörpers 5 dar, durch welche elektrische Energie von und zur Umgebung übertragen werden kann. Die Wände 9 der beiden Hydrogelkapseln 7 formen jeweils einen Teil dieser Oberfläche 13, so dass Wasser ungehindert in das Hydrogel 10 ein- bzw. austreten kann. Die beiden Elektroden 3, 4 haben einen Abstand, der vorzugsweise im Bereich von 2 bis 20 mm liegt. Anstelle einer Einschraubelektrode könnte gleichermaßen eine in anderer Form gestaltete Spitzenelektrode 3 vorgesehen sein, beispielsweise eine Spitzenelektrode in konischer Form wie sie in Fig. 5 gezeigt ist.

In der Elektrodenleitung 1 können während einer kernspintomographischen Untersuchung die Temperaturen der Spitzenelektrode 3 und/oder der Ringelektrode 4 kontinuierlich überwacht werden. Zu diesem Zweck ist es lediglich erforderlich, eine äußere Abmessung bzw. Größe der beiden Hydrogelkapseln, hier beispielsweise eine Abmessung senkrecht zur Erstreckungsrichtung der Elektrodenleitung 1, durch eine Auswertung der kernspintomographischen Aufnahmen zu bestimmen. Dabei kann die Temperatur der Spitzen- und Ringelektrode 3, 4 mittels einer einfachen Eichmessung, bei der ein Zusammenhang zwischen Größe und Temperatur der Hydrogelkapsel 7, 8 bestimmt wird, ermittelt werden.

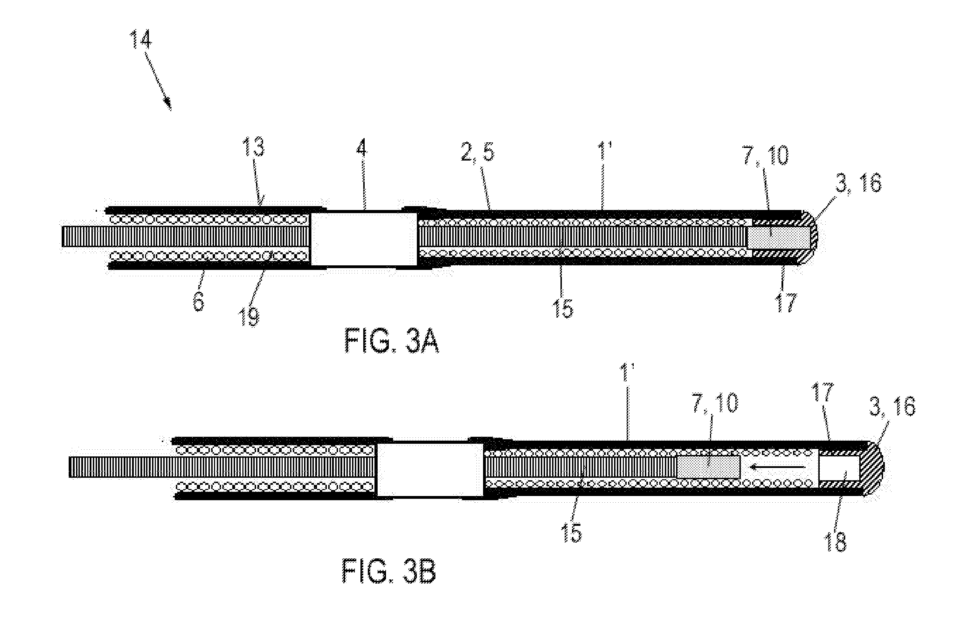

In den Figuren 3A und 3B ist ein Ausführungsbeispiel der erfindungsgemäßen Elektrodenleitungsanordnung veranschaulicht, welche insgesamt mit der Bezugszahl 14 bezeichnet ist. Die Elektrodenleitungsanordnung 14 umfasst eine herkömmliche Elektrodenleitung, welche über keine Hydrogelkapseln verfügt und insgesamt mit der Bezugszahl 1' bezeichnet ist. Die Elektrodenleitungsanordnung 14 umfasst weiterhin einen Führungsdraht 15 zum Positionieren der Elektrodenleitung 1'.

Die Elektrodenleitung 1' verfügt über einen schlauchartigen, flexiblen, elektrisch isolierten Leitungskörper 5, der mit einer in Form einer gerundeten Kappe ausgebildeten Spitzenelektrode 3 und einer proximal versetzten Ringelektrode 4 versehen ist, die jeweils elektrisch leitende Oberflächenbereiche des Leitungskörpers 4 darstellen. Die beiden Elektroden 3, 4 sind mit einer innerhalb des Leitungskörperhohlraums 19 angeordneten elektrischen Zuleitung 6 elektrisch verbunden.

Die Spitzenelektrode 3 setzt sich aus einem abstehenden Kappenabschnitt 16 und einem daran angeformten Steckabschnitt 17 zusammen, der in das distale Ende des Leitungskörpers 5 eingesteckt und fest mit diesem verbunden ist. Der Steckabschnitt 17 formt einen einseitig offenen Elektrodenhohlraum 18. Andererseits verfügt der Führungsdraht 15 an seinem distalen Ende über eine Hydrogelkapsel 7, die mit ihm fest verbunden ist. Bezüglich der Wand 9 und des in der Hydrogelkapsel 7 enthaltenen Hydrogels 10 wird auf obige Ausführungen Bezug genommen.

Wenn nun der Führungsdraht 15 in den Leitungskörperhohlraum 19 eingeführt und in Arbeitsstellung zum Positionieren der Elektrodenleitung 1' gebracht wird, dringt die Hydrogelkapsel 7 in den Elektrodenhohlraum 18 ein und ist durch Berührungskontakt mit der Spitzenelektrode 3 thermisch gekoppelt (siehe Fig. 3A). Somit kann während der Positionierung der Elektrodenleitung 1' über eine Änderung einer äußeren Abmessung der Hydrogelkapsel 7 die Temperatur der Spitzenelektrode 3 kontinuierlich überwacht werden. Der Führungsdraht 15 kann anschließend wieder entfernt werden, wobei die Hydrogelkapsel 7 aus dem Elektrodenhohlraum 18 gezogen wird (siehe Fig. 3B). In besonders vorteilhafter Weise kann bei dieser Ausgestaltung der Erfindung eine herkömmliche Elektrodenleitung 1' eingesetzt werden.

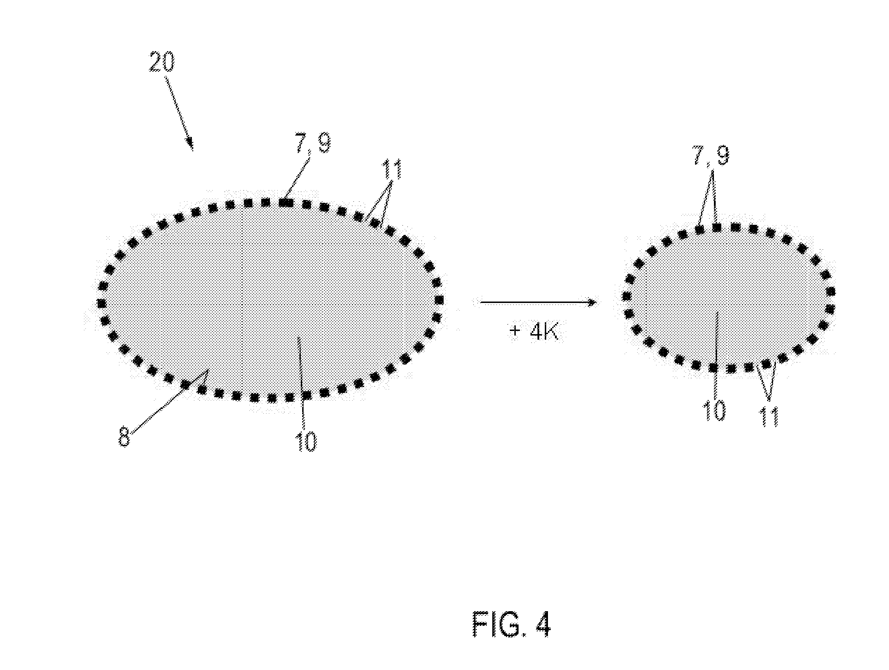

In Fig. 4 ist ein Ausführungsbeispiel des erfindungsgemäßen, implantierbaren Hydrogelsensors veranschaulicht, der insgesamt mit der Bezugszahl 20 bezeichnet ist. Demnach umfasst der Hydrogelsensor 20 eine Hydrogelkapsel 7 mit einer nachgiebigen Wand 9, die einen Kapselhohlraum 8 formt, in dem ein Hydrogel 10 enthalten ist. Die Wand 9 ist mit einer Vielzahl Löcher 11 versehen, um den Durchtritt von Wasser von/zu dem Hydrogel 10 zu ermöglichen. Bezüglich der Wand 9 und des in der Hydrogelkapsel 7 enthaltenen Hydrogels 10 wird auf obige Ausführungen Bezug genommen. Der Hydrogelsensor 22 kann implantiert werden, um die Temperatur seiner Umgebung durch Messen einer äußeren Abmessung bzw. Größe der Hydrogelkapsel 7 zu erfassen. Dies erfolgt vorzugsweise durch Auswerten einer kernspintomographischen Aufnahme. Im gezeigten Ausführungsbeispiel erfolgt eine Temperaturzunahme des implantierten Hydrogelsensors 20 um 4K, wodurch eine in der kernspintomographischen Aufnahme deutlich sichtbare Volumenreduktion der Hydrogelkapsel 7 infolge Wasserabgabe resultiert.

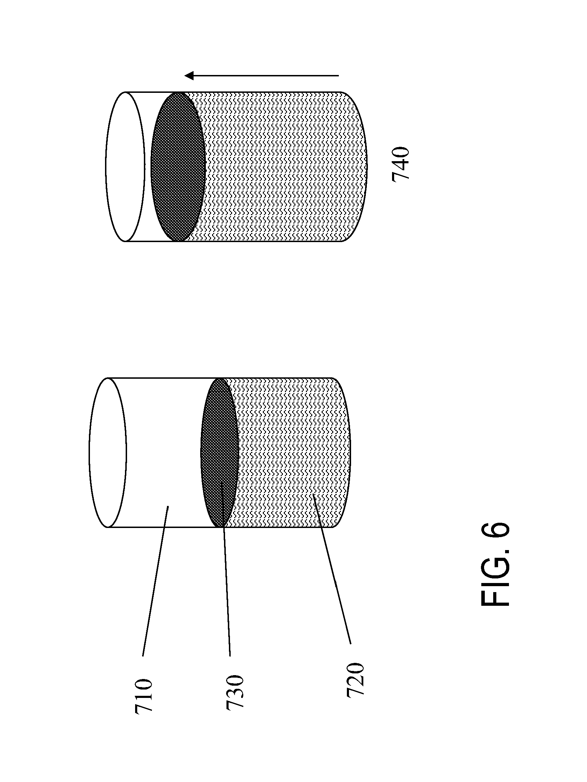

In Figur 6 ist eine Hydrogelkapsel in Kombination mit einem Wasserreservoir gezeigt. Die Elektrodenleitung bzw. der eingesetzte Führungsdraht selbst beinhaltet ein Wasserdepot 720, dass mit einer Membrane 730 oder Zuleitung mit der Hydrogelkapsel 710 verbunden ist und so die wasserdurchlässige Wand bildet, während der Rest der Hydrogelkapsel wasserundurchlässig ist. So kann abhängig von der Erwärmung der Wassergehalt zwischen Hydrogelkapsel und Wasserdepot 740 verschoben werden und per MRT-Bildauswertung das Verhältnis des Wassergehaltes entsprechend ausgewertet werden. Diese Methode bietet den Vorteil, dass dies auch in einem von der Körperflüssigkeit isoliertem Bauteil wie z.B. dem Führungsdraht eingesetzt werden kann und dass durch die Auswertung des Verhältnisses des Wasseranteils keine oder eine deutlich vereinfachte Kalibrierung benötigt wird.

Wie anhand der Ausführungsbeispiele ausführlich erläutert ist, ermöglicht die vorliegende Erfindung durch die thermische Kopplung von Elektroden mit Hydrogelkapseln eine einfache und zuverlässige Bestimmung der Temperatur der Elektroden während einer kernspintomographischen Untersuchung, so dass auch von Patienten mit implantierter Elektrodensonde kernspintomographische Aufnahmen angefertigt werden können. Erfindungsgemäßen Elektrodenleitungen bzw. Elektrodenleitungsanordnungen zeichnen sich durch eine besonders einfache Anwendung in der Praxis aus und können in der Serienfertigung einfach und kostengünstig hergestellt werden.

Darüber hinaus ermöglicht die Erfindung eine automatische Steuerung eines Kernspintomographen, wobei auf Basis der Temperatur einer oder mehrerer Elektroden einer implantierten Elektrodenleitung beispielsweise die Intensität der zur Resonanzanregung eingesetzten elektromagnetischen Wechselfelder reduziert wird, falls die Temperatur der Elektrode einen vorbestimmbaren ersten Schwellwert übersteigt. Alternativ können die elektromagnetischen Wechselfelder gänzlich abgeschaltet werden, falls die Temperatur einer Elektrode einen vorbestimmbaren zweiten Temperaturschwellwert übersteigt. Der erste und zweite Temperaturschwellwert können zueinander gleich oder voneinander verschieden sein. Gleichermaßen kann die Temperatur von einer oder mehreren Elektroden in einer graphischen Anzeigeeinrichtung angezeigt werden. Ebenso kann ein optisches und/oder akustisches Warnsignal ausgegeben werden, sobald die Temperatur einer Elektrode einen solchen Temperaturschwellwert erreicht. Denkbar wäre auch, dass eine Elektrode gezielt mit einem elektrisch Impuls beaufschlagt wird, um eine Ablation umliegenden Körpergewebes zu erreichen. Die Temperatur der Elektrode kann dabei in einfacher Weise durch die thermisch gekoppelte Hydrogelkapsel überwacht und gesteuert werden.

## Patentansprüche

1. Implantierbare Elektrodenleitung (1), welche einen distalen Abschnitt (2) mit zumindest einer Elektrode (3, 4) zur Übertragung von elektrischer Energie zwischen der Elektrodenleitung und deren Umgebung aufweist, **dadurch gekennzeichnet, dass** die Elektrodenleitung über zumindest eine mit der Elektrode (3, 4) thermisch gekoppelte Hydrogelkapsel (7) verfügt, wobei die Hydrogelkapsel zumindest eine wasserdurchlässige Wand (9) hat, die einen Hohlraum (8) formt, in dem ein Hydrogel (10) mit einem temperaturabhängigen Quellvermögen enthalten ist, wobei zumindest Teile der Wand der Hydrogelkapsel so ausgebildet ist, dass sie sich dem Volumen des Hydrogels anpassen kann.

2. Elektrodenleitung (1) nach Anspruch 1, **dadurch gekennzeichnet, dass** die Hydrogelkapsel (7) an die Elektrode (3, 4) angrenzt und sich insbesondere in Berührungskontakt mit dieser befindet.

3. Elektrodenleitung (1) nach Anspruch 1 oder 2, **dadurch gekennzeichnet, dass** die Elektrode (3, 4) eine an einem distalen Ende der Elektrodenleitung angeordnete Spitzenelektrode (3) ist.

4. Elektrodenleitung (1) nach einem der Ansprüche 1 bis 3, **dadurch gekennzeichnet, dass** die Hydrogelkapsel (7) in der Elektrodenleitung aufgenommen ist, wobei die Wand (9) der Hydrogelkapsel einen Bereich der Oberfläche (13) der Elektrodenleitung bildet.

5. Elektrodenleitung (1) nach Anspruch 4, **dadurch gekennzeichnet, dass** die Hydrogelkapsel (7) und das enthaltene Hydrogel (10) mit einem Durchgang (12) für ein Führungsmittel der Elektrodenleitung versehen sind.

6. Elektrodenleitung (1) nach einem der Ansprüche 1 bis 5, **dadurch gekennzeichnet, dass** sich das Quellvermögen des Hydrogels (10) bei steigender Temperatur verringert.

7. Elektrodenleitungsanordnung (14) zur medizinischen Anwendung, welche eine implantierbare Elektrodenleitung (1') mit einem distalen Abschnitt (2) mit zumindest einer Elektrode (3, 4) zur Übertragung von elektrischer Energie zwischen der Elektrodenleitung und deren Umgebung, sowie ein in die Elektrodenleitung (1') einführbares Führungsmittel (15) zum Führen der Elektrodenleitung umfasst,
**dadurch gekennzeichnet, dass** das Führungsmittel (15) über zumindest eine Hydrogelkapsel (7) verfügt, wobei die Hydrogelkapsel eine zumindest abschnittsweise wasserdurchlässige Wand (9) hat, die einen Hohlraum (8) formt, in dem ein Hydrogel (10) mit einem temperaturabhängigen Quellvermögen enthalten ist, wobei die Wand der Hydrogelkapsel so ausgebildet ist, dass sie sich dem Volumen des Hydrogels anpassen kann, und wobei die Hydrogelkapsel (7) so angeordnet ist, dass sie in einer zum Führen der Elektrodenleitung geeigneten Stellung des Führungsmittels (15) mit der Elektrode (3) thermisch gekoppelt ist.

8. Elektrodenleitungsanordnung (14) nach Anspruch 7, **dadurch gekennzeichnet, dass** ein Wasserdepot (720) mit einer Membrane (730) oder Zuleitung mit der Hydrogelkapsel (710) verbunden ist und so die wasserdurchlässige Wand bildet, während der Rest der Hydrogelkapsel entweder wasserdurchlässig oder wasserundurchlässig ist.

9. Elektrodenleitungsanordnung nach Anspruch 7, **dadurch gekennzeichnet, dass** die Hydrogelkapsel (7) mit einem Wasserreservoir über eine wasserdurchlässige Wand (730), wie eine Membran oder eine Leitung oder Öffnung, verbunden ist und die nicht an das Wasserreservoir angrenzenden Wände entweder wasserdurchlässig oder wasserundurchlässig sind.

10. Implantierbarer Hydrogelsensor (20), welcher eine Hydrogelkapsel (7) mit einer einen Hohlraum (8) formenden, zumindest abschnittsweise wasserdurchlässigen Wand (9) umfasst, wobei im Hohlraum (8) ein Hydrogel (10) mit einem temperaturabhängigen Quellvermögen enthalten ist, wobei die Wand so ausgebildet ist, dass sie sich dem Volumen des Hydrogels anpassen kann.

11. Verwendung des implantierbaren Hydrogelsensors (20) nach Anspruch 10 zur Bestimmung der Umgebungstemperatur des implantierten Hydrogelsensors (20).

12. Verfahren zum Bestimmen der Temperatur einer Elektrode (3, 4), welches die folgenden Schritte umfasst:
- Bereitstellen einer implantierbaren Elektrodenleitung (1), welche einen distalen Abschnitt (2) mit zumindest einer Elektrode (3, 4) zur Übertragung von elektrischer Energie zwischen der Elektrodenleitung und deren Umgebung aufweist, wobei die Elektrodenleitung über zumindest eine mit der Elektrode thermisch gekoppelte Hydrogelkapsel (7) verfügt und die Hydrogelkapsel eine zumindest abschnittsweise wasserdurchlässige Wand (9) hat, die einen Hohlraum (8) formt, in dem ein Hydrogel (10) mit einem temperaturabhängigen Quellvermögen enthalten ist, wobei die Wand der Hydrogelkapsel so ausgebildet ist, dass sie sich dem Volumen des Hydrogels anpassen kann;
- Messen einer äußeren Abmessung der Hydrogelkapsel (7);
- Ermitteln der Temperatur der Elektrode (3, 4) auf Basis der äußeren Abmessung der Hydrogelkapsel (7).

13. Verfahren nach Anspruch 12, bei welchem die äußere Abmessung der Hydrogelkapsel (7) mittels einer kernspintomographischen Aufnahme bestimmt wird.

14. Verfahren zum Betreiben eines Kernspintomographen, welches die folgenden Schritte umfasst:
- Bereitstellen einer implantierbaren Elektrodenleitung (1), welche einen distalen Abschnitt (2) mit zumindest einer Elektrode (3, 4) zur Übertragung von elektrischer Energie zwischen der Elektrodenleitung und deren Umgebung aufweist, wobei die Elektrodenleitung über zumindest eine mit der Elektrode thermisch gekoppelte Hydrogelkapsel (7) verfügt und die Hydrogelkapsel eine zumindest abschnittsweise wasserdurchlässige Wand (9) hat, die einen Hohlraum (8) formt, in dem ein Hydrogel (10) mit einem temperaturabhängigen Quellvermögen enthalten ist, wobei die Wand der Hydrogelkapsel so ausgebildet ist, dass sie sich dem Volumen des Hydrogels anpassen kann;
- Messen einer äußeren Abmessung der Hydrogelkapsel (7);
- Ermitteln der Temperatur der Elektrode (3, 4) auf Basis der äußeren Abmessung der Hydrogelkapsel (7);
wobei auf Basis der Temperatur der Elektrode (3, 4) einer oder mehrerer der folgenden Schritte ausgeführt werden:
- Reduzierung der Intensität von zur Resonanzanregung eingesetzten elektromagnetischen Wechselfeldern, falls die Temperatur der Elektrode (3, 4) einen vorbestimmbaren ersten Schwellwert übersteigt;
- Abschalten der elektromagnetischen Wechselfelder, falls die Temperatur der Elektrode (3, 4) einen vorbestimmbaren zweiten Temperaturschwellwert übersteigt;
- Darstellen der Temperatur der Elektrode (3, 4) in einer graphischen Anzeigeeinrichtung;
- Beaufschlagen der Elektrode mit einem elektrischen Impuls zum Zwecke einer Ablation umliegenden Körpergewebes.

15. Verwendung eines Kernspintomographen zum Bestimmen einer äußeren Abmessung einer mit einer Elektrode (3, 4) einer implantierbaren Elektrodenleitung (1) thermisch gekoppelten Hydrogelkapsel (7), welche eine zumindest abschnittsweise wasserdurchlässige Wand (9) hat, die einen Hohlraum (8) formt, in dem ein Hydrogel (10) mit einem temperaturabhängigen Quellvermögen enthalten ist, wobei die Wand der Hydrogelkapsel so ausgebildet ist, dass sie sich dem Volumen des Hydrogels anpassen kann.
